(19) Europäisches Patentamt
European Patent Office
Office européen des brevets

(11) **EP 2 243 490 B1**

(12) **EUROPEAN PATENT SPECIFICATION**

(45) Date of publication and mention
of the grant of the patent:
**13.06.2012 Bulletin 2012/24**

(51) Int Cl.:
*A61K 38/28* (2006.01)      *A61K 9/107* (2006.01)
*A61K 9/19* (2006.01)        *A61K 47/24* (2006.01)
*A61K 47/30* (2006.01)       *A61K 47/44* (2006.01)
*A61P 3/10* (2006.01)

(21) Application number: **08871676.6**

(22) Date of filing: **29.12.2008**

(86) International application number:
**PCT/CN2008/002112**

(87) International publication number:
**WO 2009/094846 (06.08.2009 Gazette 2009/32)**

(54) **INSULIN NASAL POWDER INHALATION**

INSULIN-NASENPULVERINHALATION

INHALATION D'UNE POUDRE À BASE D'INSULINE NASALE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **28.12.2007 CN 200710173622**
**09.04.2008 CN 200810035771**

(43) Date of publication of application:
**27.10.2010 Bulletin 2010/43**

(73) Proprietor: **Shanghai Institute of Pharmaceutical Industry**
**Shanghai 200040 (CN)**

(72) Inventors:
• **JIN, Fang**
**Shanghai 200437 (CN)**

• **LEI, Bokai**
**Shanghai 200437 (CN)**
• **WEN, Cong**
**Shanghai 200437 (CN)**

(74) Representative: **Elzaburu Marquez, Alberto**
**ELZABURU S.L.P.**
**C/ Miguel Angel, 21, 2o**
**28010 Madrid (ES)**

(56) References cited:
**WO-A1-02/064115      CA-A1- 2 215 800**
**CN-A- 1 456 351       CN-A- 1 919 337**
**CN-A- 1 919 338**

**Description**

Technical Field

The present invention relates to an insulin nasal formulation, and particularly to an inhalable nasal powder preparation of insulin, and the preparation thereof.

Background

Insulin is a polypeptide hormone consisting of 51 amino acids, which has a molecular weight of 5800 and is produced in β islet cells of normal individuals (without diabetes mellitus). Insulin plays a role in regulating glycometabolism and reducing blood glucose level; and the lack of insulin results in diabetes mellitus in an individual. Long-term and frequent administration of insulin is so necessary in many patients with diabetes mellitus as to maintain an acceptable blood glucose level.

The most common administration method of insulin is subcutaneous injection, typically in abdomen or upper thigh. To maintain an acceptable blood glucose level, at least once-a-day or twice-a-day injections of insulin is needed, while injection of immediate-release insulin may be supplemented if necessary. The administration mode of injection for insulin incurs inconvenience to a patient. First, the patients deem the frequent injection difficult and troublesome, as well as painful, which leads to decreased patient compliance, and thereby patients with severe diabetes may be endangering their lives. Additionally, insulin administered subcutaneously is absorbed slowly, typically over 45 to 90 minutes, and released slowly, which leads to increased risk for hypoglycemia. Therefore, a mode of administration which eliminates the need for self injection and enable prompt absorption of insulin *in vivo* and the corresponding insulin preparations are needed in the art.

Various potential alternative insulin administrations are reported recently, including enteric preparation for oral administration, rectal, transcutaneous, pulmonary and other preparations.

Although these techniques eliminate the discomfort associated with subcutaneous injection, they are limited by their shortages. Rectal administration is neither convenient nor comfortable, and thus is less accepted by the patients. The enteric oral formulation of insulin is most accepted by the patients, but insulin, as a polypeptide, is very susceptible to proteases inside the gastrointestinal tract, and insulin can hardly permeate the very compact mucosa of the gastrointestinal tract, and it is difficult to determine the release time of the enteric oral formulation of insulin, that is, the agent could possibly be released into the blood at an inappropriate time. Transcutaneous administration needs to bypass the barrier of skin, the bioavailability thereof, thereby, is very slow and the cost is too high. Advances have been acquired in the field of pulmonary administration, for example, pulmonary powder insulation *exubera* have been commercialized by Pfizer Inc. since 2006. Although insulin is rapidly absorbed and pain is eliminated, it did not reach the expected commercial success because dexterity is required in its use and the physiological conditions of the patients need to be examined periodically.

In the early 1990's, INS nasal formulation was studied actively, and inhalation powder was particularly focused on account of accurate dosing and the ability of increasing the stability of polypeptide agents and facilitating storage. An absorption enhancer, such as cholate, fatty acid, saponin, sodium caprylate, sodium laurate, or polyacrylic acid was used in the preliminary research. But long-term use thereof will impair the nasal mucosa and cilium, resulting in failure of the administration route. It is prerequisite to clinical use of nasal formulation of insulin to reduce or eliminate the toxicity of the active agent and its additives to the cilia, and to improve the trans-membrane absorption of insulin.

Chinese Patent Application number 01801146.2 entitled "PERNASALLY ABSORBABLE INSULIN PREPARATIONS" filed on 2001 to DDS Research Ltd. disclosed insulin preparations containing as the carrier porous spherical calcium carbonate which is an aggregate of column or needle crystallites or parallel intergrowths of both, in which the size of calcium carbonate microparticle is from 18 to 115 $\mu$m, the specific surface area thereof is no lower than 1.5 $m^2/g$, and insulin is attached to the microparticle in monolayer or multilayer. One of disadvantages of this patent is use of large amount of calcium carbonate carrier, potentially resulting in irritability to the nasal mucosa.

A Chinese Patent CN02804546.7 entitled "COMPOSITIONS FOR NASAL ADMINISTRATION OF INSULIN" filed on 2002 to Translational Research Ltd. disclosed granular compositions for nasal administration which comprise aggregated crystalline cellulose having a specific particle distribution as a carrier for insulin, in which insulin powder has not been solubilised, and more than 90% of the aggregated crystalline cellulose has a particle diameter from 10 to 350 $\mu$m; the weight ratio between the insulin powder and the aggregated crystalline cellulose is from 1:1 to 500, and preferably from 1:2 to 100. The composition is prepared by mixing the powder of the agent and the solid carrier well. One of disadvantages of this patent is use of large amount of crystalline cellulose, and addition of insoluble materials will potentially result in irritability to the nasal mucosa.

Chinese Patent Application number 200480041300.9 entitled "PHARMACEUTICAL COMPOSITIONS AND METHODS FOR INSULIN TREATMENT" filed on 2004 to Bentley Pharmaceuticals, Inc. disclosed compositions and methods for treating a patient with insulin that combines insulin, a permeation enhancer, and a liquid carrier that maintains an acidic pH of no greater than 4.5, and it is delivered as a nasal spray. The permeation enhancer is CPE-215 (cyclopentadecalactone), which is prone to crystallize under a lower temperature. Thus, to maintain the stability of insulin, the formulation needs to be cryopreserved, whereby addition of crystallization inhibitor is needed. Further, the permeation enhancer is emulsified in an aqueous phase containing insulin, and thereby a surfactant with HLB from 7 to 14 is selected, and the size of the resulting oil droplets is from 0.1 to 20 $\mu$m. Therefore, one of disadvantages of this patent is the potential irritability and toxicity of the permeation enhancer to the nasal mucosa, and being readily absorbed *in vivo* may lead to unexpected side effects.

Some Chinese Patent Applications concerning correlated technologies had been filed by the present inventor, i.e., Chinese Patent Application number 200510028990.0 entitled "Insulin nasal powder and preparation thereof" and Chinese Patent Application number 200510028991.5 entitled "A liquid formulation for nasal administration of insulin".

Microemulsion system is used in both of the above applications, to increase the lipophilicity of insulin molecules. Bioadhesive materials are also used, to increase the retention of self-microemulsified powder containing insulin in nasal cavity. A powder formulation of insulin is produced by lyophilizing the agent-containing microemulsion followed by mixing with an appropriate carrier in Chinese Patent Application number 200510028990.0. In both of the above applications, most of the agent-containing microemulsions have a particle diameter of greater than 100 nm, and large amount of surfactant is needed when preparing a microemulsion having a particle diameter of less than 100 nm, which makes the ratio between non-phospholipid surfactant (poloxamer) and phospholipid up to 3:1-5:1. It was discovered through experiments that when lecithin and poloxamer are used as emulsifiers, the physical stability of the resulting system is low due to the large difference of HLB therebetween (HLB is from 5 to 6 for lecithin, and 25 for poloxamer). Despite excellent safety profiles and wide applications in preparation of fat emulsion, the resulting emulsions generally have a particle diameter of greater than 100 nm. Decreased particle diameter may reduce the usage of oil and thereby reduce cost, and improved physical stability facilitates the chemical stability and reduces the activity loss of agents.

Meanwhile, cosurfactant, such as ethanol, ethylene glycol, and propylene glycol, is used in the formulations of the above applications. It is well known in the art that addition of cosurfactant facilitates formation of microemulsion. Addition of cosurfactant however influences the activity of insulin, and in turn, reduces the efficacy and increases the medicinal expenses for patients.

Accordingly, selection of surfactant and other adjuvants, optimization of the ratio between insulin and surfactant/other adjuvants in the insulin nasal formulation, and prevention of hypoglycemia and reduction of irritability and toxicity to nasal mucosa have always been the interesting subjects in the art.

Summary of the Invention

The technical problem to be solved by the present invention is to provide an inhalable nasal powder preparation of insulin to overcome the above deficiencies in the prior art, and to meet the needs in clinic.

The technical concept of the invention is:

The invention provides a nasal formulation as defined in the claims, and thereby avoids the undesirable physiological influences on lung typically associated with the pulmonary administration and the potential adverse effects of injection. The nasal mucosa has a large surface area (from 150 to 180 cm$^2$) with a large amount of cilia attached thereto, which means an increased effective area for absorption of agents. The nasal mucosa is thin (2 ~ 4 mm) and abundant in blood and lymphatic capillaries underneath the epithelial cells, which means that the drug can be absorbed more rapidly. And, there are less enzymes of degradation in the nasal cavity than in the gastrointestinal tract, which means less damage to polypeptide agents. Therefore, the nasal formulation of the invention can provide various advantages, such as lower dosage, higher availability and rapid onset of action. Also, the powder formulation of the invention has many advantages over a liquid formulation of insulin, including the elimination of preservatives, reduced risk of nasal irritation, elongated retention of the agent in the nasal cavity, and enhanced trans-membrane absorption of insulin due to the enrichment of the powder near the cilia-dense olfactory area after the active inhalation.

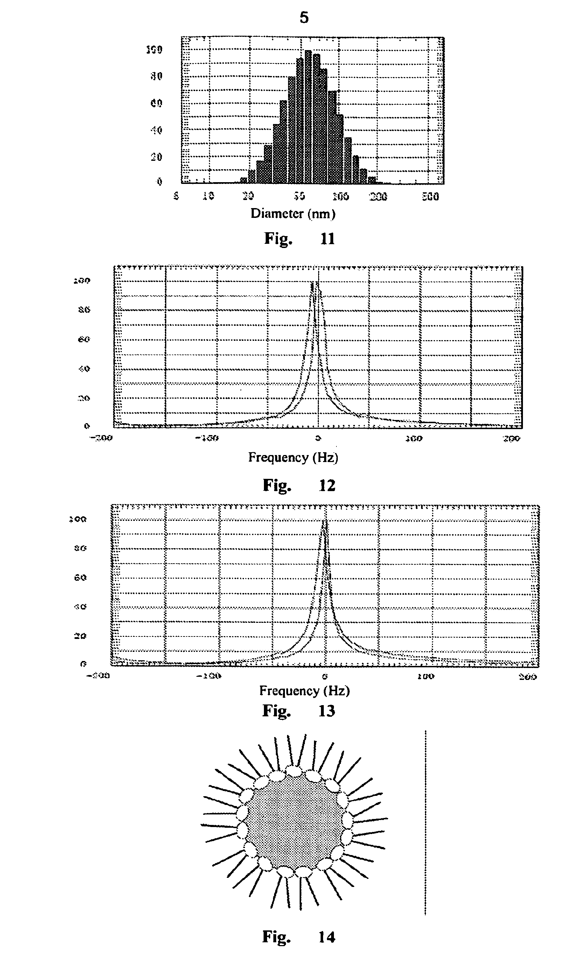

In view of the fact that both hydrophilic chains and hydrophobic chains are present in an insulin molecule, the present inventors prepared a emulsion containing insulin as an active ingredient, water as a continuous phase and lipid as a disperse phase, which is referred to as an oil-in-water emulsion (O/W). Experiments show that insulin, as an amphiphilic protein, is soluble in the aqueous phase of the emulsion, and the hydrophobic core of the dimer has an interaction of adsorption with the lipid phase. In the oil-in-water emulsion, the lipid phase is suspended in the aqueous phase as spheric or nearly spheric oil droplets, with the hydrophobic core of the dimer of insulin adsorbed to the surface of each, as shown in figure 14.

The present invention finds that, as to the prepared microemulsion containing the drug agent, a coordinated relationship between the amount of insulin as the active ingredient and the surface area of the oil droplets is important. Simply increasing the amount of insulin may not improve the therapeutic efficacy but increase the cost. Based on these findings, the present invention provides:

An inhalable nasal powder preparation of insulin comprises a self-microemulsifiable powder with therapeutically effective amount of insulin and a pharmaceutically acceptable carrier. Preferably, the formulation comprises the components and amounts by weight percentages of:

| | |
|---|---|
| self-microemulsifiable Lyophilized powder of insulin Carrier | 1 ~ 100%, and 0 ~ 99%; |
| More preferably, self-microemulsifiable Lyophilized powder of insulin Carrier | 15 ~ 80%, and 20 ~ 85%; |
| Even more preferably, self-microemulsifiable Lyophilized powder of insulin Carrier | 15 ~ 60%, and 40 ~ 85%. |

The carrier may be mixture of one or more selected from the group consisting of mannitol, lactose, microcrystalline cellulose and chitosan.

The self-microemulsifiable Lyophilized powder of insulin comprises insulin, lipid, emulsifier, antioxidant, supporting agent, bio-gel adhesive material and pH modifier.

For each gram (1g) of insulin, the formulation may further include:

| | |
|---|---|
| antioxidant | 0.005 ~ 0.045 g, and preferably 0.015 ~ 0.03 g; |
| supporting agent | 0 ~ 5 g, and preferably 1.0 ~ 2.0 g; |
| bio-gel adhesive | 0.01 ~ 1 g, and preferably 0.1 ~ 0.5 g; and |
| pH modifier | 0.0 1 ~ 1.0 g, and preferably 0.02 ~ 0.5 g;. |

Wherein, the amount of lipid can be determined according to the following equations:

the surface area of the hydrophobic core of insulin : the surface area of the oil droplet = 1 : 0.5~2, and preferably 1 : 1~1.5, wherein the average diameter of the oil droplets is about 20 to 200 nm, and preferably 40 to 100 nm; the ratio (w/v) of lipid: emulsifier = 1 ml : 0.6~ 1.2 g, and preferably 1 : 0.78~ 1.1 g; and when the said emulsifier is a mixture of lecithin and Tween 80, the weight ratio of lecithin : Tween 80 = 1 : 0.10 ~ 0.4, and preferably 1 : 0.2 ~ 0.33.

The said self-microemulsifiable Lyophilized powder of insulin has a diameter of about 10 to 150 $\mu$m, which can be prepared by vacuum lyophilization or spray drying of a nanoemulsion of insulin.

The said supporting agent is selected from the group consisting of lactose, mannitol, xylitol, sorbitol and their combinations, with mannitol being preferred.

For each gram (1g) of insulin, the said nanoemulsion may further include:

| | |
|---|---|
| antioxidant | 0.005 ~ 0.045 g, preferably 0.015 ~ 0.03 g; |
| supporting agent | 0 ~ 5 g, preferably 1.0 ~ 2.0 g; |
| bio-gel adhesive | 0.0 1 ~ 1g, preferably 0.1 ~ 0.5 g; |
| pH modifier | 0.01 ~ 1.0 g, preferably 0.02 ~ 0.5 g; and |
| water | 6 ~ 96 ml; |

Wherein, the amount of lipid is determined according to the following equations:

the total surface area of the hydrophobic core of insulin : the total surface area of the oil droplet = 1 : 0.5~2, and preferably 1 : 1~1.5; and the average diameter of the oil droplets can be about 20 to 200 nm, and preferably about 40 to 100 nm; the ratio (v/w) of lipid : emulsifier = 1 ml : 0.6~1.2 g, and preferably 1 : 0.78~1.1 g; when the said emulsifier is a mixture of lecithin and Tween 80, the weight ratio of lecithin : Tween 80 is 1 : 0.10 ~ 0.35, and preferably 1 : 0.2 - 0.33.

The surface area of the hydrophobic core of insulin has been well defined. As reported (see for example The peptides Analysts, synthesis, Biology, 1981, 4: 63), the said surface area of each hydrophobic core is calculated to be about 1.5 nm$^2$, each gram (1 g) of insulin contains about $1.03 \times 10^{20}$ molecules, which corresponds to about $5.15 \times 10^{19}$ dimers, and therefore, the total surface area of the hydrophobic cores in the gram is about $7.7 \times 10^{19}$ nm$^2$. That is, for each gram of insulin, the hydrophobic cores of insulin can offer an active surface of about $7.7 \times 10^{19}$ nm$^2$ for the interaction of adsorption with the oil droplets.

For example, the particle diameter of the oil droplets can be determine using Nicomp-380 laser granulometer according to a previously described method (see, for example, Application of dynamic light scattering on the evaluation of the particles size and distribution of submicroemulsion for injection, ZHENG Shaohui, DENG Yihui, Chinese Journal of Pharmaceutics, 2005, 3(3): 126).

Specifically, the surface area of an individual oil droplet is can be calculated as follows :

The surface area droplet $S_1=4\pi r^2=\pi d^2$;

The volume of droplet $V_1 = \frac{4}{3}\pi r^3 = \frac{\pi d^3}{6}$; ;

Then, when the lipid of a total volume of V is dispersed into oil droplets with a diameter of d in the system, the total number of oil droplets is n = $V/V_1$;

the total surface area of oil droplets is n $\times$ $S_1$; and

for each unit of dispersed lipid, the specific surface area is $\delta = nS_1 = \frac{S_1}{V_1} = \frac{6}{d}$.

The said lipid can be, for example, a synthetic lipid such as decanoyl and octanoyl glycerides, a natural lipid of a plant origin such as soy, coconut, tea or peanut or an animal origin such as fish, or their combination. Preferred is soy oil, tea oil, decanoyl and octanoyl glycerides, or their combinations. More preferred is decanoyl and octanoyl glycerides.

The said supporting agent is selected from lactose, mannitol, xylitol, sorbitol, or their combinations, with mannitol being preferred

The said bio-gel adhesive is selected from chitosan, alginate, gum arabic, hydroxypropyl methylcellulose, hydroxypropyl cellulose, sodium carboxymethyl cellulose, or their combinations. Preferred is chitosan, sodium alginate, or their combination. More preferred is chitosan.

The said pH modifier can be, for example, HCl, NaOH, an organic acid, an organic base, or their combinations. The organic acid includes, but is not being limited to, acetic acid, citric acid, glycine, or arginine, and the organic base includes, but is not limited to, trometamol and ethanolamine, etc. Preferably, the pH modifier is selected from the group consisting of HCl, NaOH, citric acid, glycine and their combiantions.

Preferably, the said self-microemulsifiable powder of insulin contains:

| | |
|---|---|
| insulin | 1 g; |
| antioxidant | 0.005 ~ 0.045 g, preferably 0.015 ~ 0.03 g; |
| supporting agent | 0 ~ 5 g, preferably 1.0 ~ 2.0 g; |
| bio-gel adhesive | 0. 01 ~ 1 g, preferably 0.1 ~ 0.5 g; |
| pH modifier | 0.01 ~ 1.0 g, preferably 0.02 ~ 0.5 g; |
| lipid | 0.13 ~ 2.6 ml, preferably 0.51 ~ 1.3 ml; |
| emulsifier | 0.24 ~ 3.0 g, preferably 0.4 ~ 1.4 g; |

Wherein, the average diameter of oil droplets is preferably 40 to 100 nm;

The said antioxidant is preferably one or more selected from the group consisting of ascorbyl palpitate, t-butyl p-hydroxyanisole, propyl gallate and vitamin E, with vitamin E being preferred.

In the present invention, the amount of lipid is determined according to the surface area and the diameter of the oil droplets. Plentiful experiments in animal prove that best therapeutic efficacy is obtainable with the formulations as specified above.

The nasal formulation of insulin according to the present invention can be prepared by a method including:

Preparation of a nanoemulsion of insulin, including the steps of:

(1) dissolving a bio-gel adhesive in water or a HCl solution to produce a mixture of the bio-gel adhesive and water or the HCl solution;

(2) mixing the lipid, surfactant, and antioxidant to dissolve, mixing the obtained lipid phase with water and stirring into a primary emulsion, homogenizing the obtained primary emulsion in emulsion homogenizer for 2 ~ 10 cycles under a pressure between about 400 - 800 bar to produce a semitransparent or transparent microemulsion, wherein the diameter of oil droplets is about 20 to 200 nm, and preferably 40 to 100 nm;

(3) Mixing a specified amount of insulin with the microemulsion obtained in step (2) and the hydrogel containing mixture of step (I), and adding an acid, for example, HCl (1-6 mol/L), to dissolve insulin;

(4) Adding mannitol, and adjusting pH to 3.5 - 8.5, to obtain a nanoemulsion of insulin;

Preparation of a self microemulsifiable powder of insulin, including the step of:

Subjecting the nanoemulsion of insulin obtained above to lyophilizing or spray-drying, and sieving for the particles having a size of about 10 to 150 $\mu$m as the self-microemulsifiable powder of insulin.

The said lyophilization may run through the stages including sequentially:

-40 °C~ -30 °C for 3 - 4 hours, -15 °C~ -10 °C for 10 ~ 15 hours, -5 °C- 0 °C for 2 - 4 hours, 5 °C- 10 °C for 2 - 4 hours and 20 °C- 25 °C for 1 - 2 hours;

For the said spray drying, the inlet temperature may be 80 to 100 °C, and the outlet temperature 50 to 80 °C;

And, preparation of the inhalable nasal powder preparation of insulin:

Mixing a specified amount of the self-microemulsifiable powder of insulin obtained above with a carrier, filling the mixture into a suitable container, such as capsule, vesicle or other appropriate nasal inhalation devices, to obtain a unite package containing 0.5 - 5 mg insulin.

The insulin nasal formulation of the invention is administered by active inhalation. The formulation can be used to treat insulin-dependent diabetes. The dose is typically 25 to 100 IU, which can be readily determined by a physician considering the relevant factors including the patient's condition, e.g., the severity of the disease.

The present invention finds in animals experiments that the hypoglycemic effect is correlated to the amount of oil and the particle diameter of oil droplets, and the particle diameter of oil droplets is correlated to the selection of emulsifier and its amount. Typically, the more the emulsifier, the smaller the oil droplets, while undesirably, the lower the safety. Therefore, all of these factors should be taken into consideration to select appropriate emulsifiers and their amount.

The inventors determined the amount of emulsifier using the toad palate ciliotoxicity assay. When the concentration of Tween was no more than 5%, no substantial cilium toxicity was observed. Further, even if the concentration of Tween is below 5%, by introducing phospholipid at an appropriate ratio of Tween/phospholipid, it can still produce an emulsion with a particle size of less than 100 nm, which facilitates the transmucosal absorption of insulin. It is demonstrated in experiments that the microemulsion system of the invention has a good physical stability and an improved mucosal safety. Plentiful experiments have shown that there exists a proportional relationship between the amount of lipid and the amount of insulin; and, the hypoglycemic effect can be observed when the ratio of the surface area of the hydrophobic cores of insulin to the surface area of the oil droplets is or above 1:0.5, the best result may be observed at a ratio of about 1: 1~1.5, and a further increased ratio does not improve the hypoglycemic effect but may potentially increase the mucosal irritation and ciliotoxicity.

The inventors discovered that lyophilization or spray-drying, under controlled condition and parameters, can make the agent-containing microemulsion solidified to give a self-microemulsifiable powder with no harm to the therapeutic efficacy. The stability of insulin can be improved by a drying process.

The present invention successfully established the correlation between the amount of insulin and the oil droplets, and selected the suitable emulsifier(s) to obtain a nanoemulsion of insulin. One advantage of the invention is the omission of cosurfactant in the formulation. With the formulation of the invention, the alcohols' influence on the activity of insulin is eliminated, the therapeutic efficacy is improved, and the patients' medication expenses are decreased. The solid powder with self-microemulsifying property, obtained by lyophilization or spray drying, is much more stable than a liquid formulation. The powder can be regenerated into a nanoemulsion in water. After the agent-containing composition is administered to the nasal cavity, the nanoemulsion permeates more easily through the barrier of nasal mucosa into the blood and lymphatic capillaries, which lead to an improved bioavailability and absorption *in vivo,* with no irritation to the nasal mucosa. And, the use of a bioadhesive increases the retention of the drug-containing powder on the nasal mucosa, allowing for a more complete uptake and action of the drug.

Brief Description of the Drawings

Figure 1 shows the results of an *in vitro* insulin transmucosal assay.

Figure 2 shows the results of a hemolysis assay.

Figure 3 shows the average blood glucose curves obtained in rabbits after nasal administration with an insulin powder, the inhalable powder of insulin and a regenerated solution thereof.

Figure 4 shows the average blood glucose curve obtained in rabbits after nasal administration with different inhalable powders of insulin in the examples.

Figure 5 shows the area over curve of blood glucose versus the ins-lipid ratio.

Figure 6 shows the average blood glucose curve obtained in Beagle dogs after nasal administration of insulin.

Figure 7 the average blood glucose curve in Beagle dogs in a pharmacokinetic assay.

Figure 8 plots the average concentration of drug against time for a subcutaneous administration and a nasal administration.

Figure 9 shows the pathological section of the nasal mucosa of rabbit.

Figure 10 shows the particle diameter of a blank microemulsion (Example 9) (average particle diameter: 61.4 nm).

Figure 11 shows the particle diameter of the insulin nanoemulsion (1 : 0.8) (average particle diameter: 66.3 nm).

Figure 12 shows the zeta-potential of a blank microemulsion (Example 9) (-17.20 mv).

Figure 13 shows the zeta-potential of the insulin nanoemulsion (1 : 0.8) (-7.54 mv).

Figure 14 is a diagram of the hydrophobic cores of insulin dimers adsorbed on the surface of an oil droplet.

<u>Preferred Embodiments of the Invention</u>

Example 1

75 mg chitosan was dissolved in 1 ml of 0.1 mol/l HCl.

1.6 g lecithin, 2.0 ml decanoyl and octanoyl glycerides, 0.4 g Tween 80, and 40 mg vitamin E were dissolved at 60 °C to obtain a lipid phase.

To the lipid phase at 60 °C was added distilled water at the same temperature, stirred to produce a primary emulsion. The primary emulsion was further homogenized for 6 cycles under a pressure of 800 bar in a high-pressure emulsion homogenizer to obtain a blank microemulsion (60 ml), in which the average particle diameter of oil droplets was about 40 nm.

The obtained microemulsion was mixed with 3.0 g insulin, to which the above chitosan solution was added, and 0.3 ml of 3 mol/l HCl was added by dripping to dissolve insulin. 3.0 g mannitol was then added. The system was adjusted to pH 4.0 using 7.5 ml of 0.1 mol/l NaOH, and lyophilized to obtain a dry powder from the insulin nanoemulsion. The lyophilization procedures included -40 °C for 4h, -15 °C for 12h, -5 °C for 4h, 5 °C for 4h and 20 °C for 2h. The lowest eutectic point was determined as -9.4 °C. The powder was sieved and mixed with an 1:1 (by weight) mixture of mannitol and microcrystalline as the carrier, the weight ratio of the lyophilized powder to the carrier being 1:1. Thereafter, the mixture was filled into capsules , e.g., a vesicle, contain 1.5 mg insulin in each unit package as the product of the insulin nasal formulation of the invention.

Example 2

75 mg chitosan was dissolved in 1 ml of 0.1 mol/l HCl.

1.6 g lecithin, 2.0 ml decanoyl and octanoyl glycerides, 0.4 g Tween 80, and 40 mg vitamin E were dissolved at 60 °C to obtain a lipid phase.

To the lipid phase at 60 °C was added distilled water at the same temperature, stirred to produce a primary emulsion. The primary emulsion was further homogenized for 6 cycles under a pressure of 600 bar in a high-pressure emulsion homogenizer to obtain a blank microemulsion (60 ml), in which the average particle diameter of oil droplets was about 60 nm.

The obtained microemulsion was mixed with 3.0 g insulin, to which the above chitosan solution was added, and 0.3 ml of 3mol/l HCl was added by dripping to dissolve insulin. 3.0 g mannitol was then added. The system was adjusted to pH 4.0 using 7.5 ml of 0.1 mol/l NaOH, and lyophilized to obtain a dry powder from the insulin nanoemulsion. The lyophilization procedures included -40 °C for 4h, -15 °C for 12h, -5 °C for 4h, 5 °C for 4h and 20 °C for 2h. The lowest eutectic point was determined as -9.6 °C. The powder was sieved and mixed with an 1:1 (by weight) mixture of mannitol and microcrystalline as the carrier, the weight ratio of the lyophilized powder to the carrier being 1:1. Thereafter, the mixture was filled into capsules, e.g., a vesicle, contain 1.5 mg insulin in each unit package as the product of the insulin nasal formulation of the invention.

Example 3

75 mg chitosan was dissolved in 1 ml of 0.1 mol/l HCl.

1.6 g lecithin, 2.0 ml decanoyl and octanoyl glycerides, 0.4 g Tween 80, and 40 mg vitamin E were dissolved at 60 °C to obtain a lipid phase.

To the lipid phase at 60 °C was added distilled water at the same temperature, stirred to produce a primary emulsion. The primary emulsion was further homogenized for 6 cycles under a pressure of 400 bar in a high-pressure emulsion homogenizer to obtain a blank microemulsion (60 ml), in which the average particle diameter of oil droplets was about 100 nm.

The obtained microemulsion was mixed with 3.0 g insulin, to which the above chitosan solution was added, and 0.3 ml of 3mol/l HCl was added by dripping to dissolve insulin. 3.0 g mannitol was then added. The system was adjusted to pH 4.0 using 7.5 ml of 0.1 mol/l NaOH, and lyophilized to obtain a dry powder from the insulin nanoemulsion. The lyophilization procedures included -40 °C for 4h, -15 °C for 12h, -5 °C for 4h, 5 °C for 4h and 20 °C for 2h. The lowest eutectic point was determined as -9.8 °C. The powder was sieved and mixed with an 1:1 (by weight) mixture of mannitol and microcrystalline as the carrier, the weight ratio of the lyophilized powder to the carrier being 1:1. Thereafter, the mixture was filled into capsules, e.g., a vesicle, contain 1.5 mg insulin in each unit package as the product of the insulin nasal formulation of the invention.

Example 4

2 g sodium alginate was dissolved in 20 ml water.

1.2 g lecithin, 1.8 ml refined soy oil, 0.32 g Tween 80, and 30 mg vitamin E were dissolved at 60 °C to obtain a lipid phase.

To the lipid phase at 60 °C was added distilled water at the same temperature, stirred to produce a primary emulsion. The primary emulsion was further homogenized for 2 cycles under a pressure of 800 bar in a high-pressure emulsion homogenizer to obtain a blank microemulsion (40 ml), in which the average particle diameter of oil droplets was about 60 nm.

The obtained microemulsion was mixed with 2.0 g insulin, to which the above sodium alginate solution was added, and 1 mol/l HCl was added by dripping to dissolve insulin. 4 g sorbitol was then added. The system was adjusted to pH 4 using 10 ml of 0.1 mol/l NaOH, and lyophilized to obtain a dry powder from the insulin nanoemulsion. The lyophilization procedures included-30 °C for 3h, -15 °C for 10h, -5 °C for 2h, 10 °C for 2h, 25 °C for 1h.. The lowest eutectic point was determined as -10.2 °C. The powder was sieved and mixed with an 1:5 (by weight) mixture of mannitol and microcrystalline as the carrier, the weight ratio of the lyophilized powder to the carrier being 15:85. Thereafter, the mixture was filled into capsules, e.g., a vesicle, contain 5 mg insulin in each unit package as the product of the insulin nasal formulation of the invention.

Example 5

0.1 mg chitosan was dissolved in 5 ml of 0.1 mol/l HCl.

2.7 g lecithin, 2.7 ml fish oil, 0.27 g Tween 80, and 20 mg vitamin E were dissolved at 60 °C to obtain a lipid phase.

To the lipid phase at 60 °C was added distilled water at the same temperature, stirred to produce a primary emulsion. The primary emulsion was further homogenized for 10 cycles under a pressure of 400 bar in a high-pressure emulsion homogenizer to obtain a blank microemulsion (50 ml), in which the average particle diameter of oil droplets was about 200 nm.

The obtained microemulsion was mixed with 2.0 g insulin, to which the above chitosan solution was added, and 0.4 ml of 1 mol/l citric acid was added by dripping to dissolve insulin. 5 g mannitol was then added. The system was adjusted to pH 3.5 using 0.5 g glycine and spray-dried at an inlet temperature of 80 °C and an outlet temperature of 50 °C to obtain the powder. The powder was sieved and mixed with microcrystalline as the carrier in a ratio of 80:20. Thereafter, the mixture was filled into capsules, e.g., a vesicle, contain 2.5 mg insulin in each unit package as the product of the insulin nasal formulation of the invention.

Example 6

1 g hydroxypropyl cellulose was allowed to swell in 10 ml water.

2.5 g lecithin, 5.1 ml decanoyl and octanoyl glycerides, 0.5 g Tween 80, and 60 mg vitamin E were dissolved at 60 °C to obtain a lipid phase.

To the lipid phase at 60 °C was added distilled water at the same temperature, stirred to produce a primary emulsion. The primary emulsion was further homogenized for 10 cycles under a pressure of 800 bar in a high-pressure emulsion homogenizer to obtain a blank microemulsion (120 ml), in which the average particle diameter of oil droplets was about 40 nm.

The obtained microemulsion was mixed with 10.0 g insulin, to which the above hydroxypropyl cellulose solution was added, and 0.4 ml of 6 mol/l HCl was added by dripping to dissolve insulin. 20 g mannitol was then added. The system was adjusted to pH 6.0 using 5 g trometamol, and lyophilized to obtain a dry powder from the insulin nanoemulsion. The lyophilization procedures included -40 °C for 4h, -10 °C for 12h, 0 °C for 4h, 5 °C for 2h and 20 °C for 2h. The lowest eutectic point was determined as -8.8 °C. The powder was sieved and mixed with an 1:5 (by weight) mixture of mannitol and microcrystalline as the carrier, the weight ratio of the lyophilized powder to the carrier being 75:25. Thereafter, the mixture was filled into capsules, e.g., a vesicle, contain 3 mg insulin in each unit package as the product of the insulin nasal formulation of the invention.

Example 7

0.5 g carboxymethyl cellulose was dissolved in 10 ml water.

1.8 g lecithin, 2.0 ml decanoyl and octanoyl glycerides, 0.6 g Tween 80, and 60 mg vitamin E were dissolved at 60 °C to obtain a lipid phase.

To the lipid phase at 60 °C was added distilled water at the same temperature, stirred to produce a primary emulsion. The primary emulsion was further homogenized for 4 cycles under a pressure of 600 bar in a high-pressure emulsion homogenizer to obtain a blank microemulsion (40 ml), in which the average particle diameter of oil droplets was about 50 nm.

The obtained microemulsion was mixed with 2.0 g insulin, to which the above carboxymethyl cellulose solution was added, and 0.4 ml of 2 mol/l HCl was added by dripping to dissolve insulin. 4 g mannitol was then added. The system was adjusted to pH 8.5 using 0.1 mol/l NaOH and citric acid, and lyophilized to obtain a dry powder from the insulin nanoemulsion. The lyophilization procedures included -40 °C for 4h, -15 °C for 15h, -5 °C for 2h, 5 °C for 2h and 20 °C for 2h. The lowest eutectic point was determined as -9.2 °C. The powder was sieved and mixed with an 1:1:1 (by weight) mixture of mannitol, lactose, and microcrystalline as the carrier, the weight ratio of the lyophilized powder to the carrier being 1:99. Thereafter, the mixture was filled into capsules, e.g., a vesicle, contain 0.5 mg insulin in each unit package as the product of the insulin nasal formulation of the invention.

Example 8

200 mg chitosan was dissolved in 10 ml of 0.1 mol/l HCl.

13.5 g lecithin, 19.2 ml tea oil, 1.5 g Tween 80, and 450 mg vitamin E were dissolved at 60 °C to obtain a lipid phase.

To the lipid phase at 60 °C was added distilled water at the same temperature, stirred to produce a primary emulsion. The primary emulsion was further homogenized for 6 cycles under a pressure of 600 bar in a high-pressure emulsion homogenizer to obtain a blank microemulsion (75 ml), in which the average particle diameter of oil droplets was about 100 nm.

The obtained microemulsion was mixed with 10.0 g insulin, to which the above chitosan solution was added, and 0.4 ml of 6 mol/l HCl was added by dripping to dissolve insulin. 10 g mannitol was then added. The system was adjusted to pH 7.0 using 1.5 ml of 1 mol/l NaOH, and spray-dried at an inlet temperature of 120 °C and an outlet temperature of 80 °C. The powder was sieved and mixed with mannitol in a ratio of 40:60. Thereafter, the mixture was filled into capsules, e.g., a vesicle, contain 2 mg insulin in each unit package as the product of the insulin nasal formulation of the invention.

Example 9

200 mg chitosan was dissolved in 10 ml of 0.1 mol/l HCl.

5 g lecithin, 10.0 ml decanoyl and octanoyl glycerides, 2 g Tween 80, and 150 mg vitamin E were dissolved at 60 °C to obtain a lipid phase.

To the lipid phase at 60 °C was added distilled water at the same temperature, stirred to produce a primary emulsion. The primary emulsion was further homogenized for 10 cycles under a pressure of 600 bar in a high-pressure emulsion homogenizer to obtain a blank microemulsion (60 ml), in which the average particle diameter of oil droplets was about 60 nm.

The obtained microemulsion was mixed with 10.0 g insulin, to which the above chitosan solution was added, and 0.4 ml of 6 mol/l HCl was added by dripping to dissolve insulin. 10 g mannitol was then added. The system was adjusted to pH 4.5 using 4 g glycine, and lyophilized to obtain a dry powder from the insulin nanoemulsion. The lyophilization procedures included -40 °C for 4h, -15 °C for 14h, -5 °C. for 2h, 5 °C for 2h and 20 °C for 2h. The lowest eutectic point was determined as -9.0 °C. The powder was sieved and mixed with an 1:1:0.01 (by weight) mixture of mannitol, microcrystalline, and chitosan as the carrier, the weight ratio of the lyophilized powder to the carrier being 1:1. Thereafter, the mixture was filled into capsules, e.g., a vesicle, contain 1 mg insulin in each unit package as the product of the insulin nasal formulation of the invention.

Example 10

5 g hydroxypropyl cellulose was allowed to swell in 50 ml water.

2 g lecithin, 4 ml decanoyl and octanoyl glycerides, 0.4 g Tween 80, and 50 mg vitamin E were dissolved at 60 °C to obtain a lipid phase.

To the lipid phase at 60 °C was added distilled water at the same temperature, stirred to produce a primary emulsion. The primary emulsion was further homogenized for 10 cycles under a pressure of 800 bar in a high-pressure emulsion homogenizer to obtain a blank microemulsion (100 ml), in which the average particle diameter of oil droplets was about 20 nm.

The obtained microemulsion was mixed with 10.0 g insulin, to which the above hydroxypropyl cellulose solution was added, and 0.6 ml of 3 mol/l HCl was added by dripping to dissolve insulin. 25 g mannitol was then added. The system was adjusted to pH 4.5 using 5 g glycine, and lyophilized for 30h to obtain a dry powder from the insulin nanoemulsion. The lyophilization procedures included -40 °C for 4h, -15 °C for 104h, -5 °C for 4h, 5 °C for 2h and 20 °C for 2h. The lowest eutectic point was determined as -9.3 °C. The powder was sieved and mixed with mannitol in a weight ratio of 60:40. Thereafter, the mixture was filled into capsules, e.g., a vesicle, contain 1 mg insulin in each unit package as the product of the insulin nasal formulation of the invention.

Example 11

Trans-membrane Penetration

1. Methods: Fresh bovine nasal mucosa was washed with normal saline, and was cleared of the adherent tissues, such as fat. The nasal mucosa was cut into suitable size and mounted between the donor compartment and receptor compartment of a modified Franz diffusion cell, with the outer surface of the mucosa facing the donor compartment. The receptor compartment was filled with normal saline at pH 4.0 to full, and the volume of addition was recorded. The added saline was continuously stirred using a magnetic stirrer, and the compartment was kept at 37°C using an external water bath. Drug powder was evenly spread on the mucosa in the donor compartment, and was wetted by spraying saline. At different time points, 0.3 ml receptor solution was sampled, and was compensated with 0.3 ml fresh saline with a pH adjusted to 4.0. The sample was centrifuged, and the trans-membrane penetration of insulin was quantified by HPLC.

2. High-Performance Liquid Chromatography

Instrument: SHIMADZU SCL-10A VP, SPD-10A VP, LC-10AD VP, SIL-10AD VP, CTO-10AS; chromatography column: C18 250 mm × 4.6 mm (5 μm) (Dikma, Diamonsil™); mobile phase: 0.2 mol/L sulfate buffer-acetonitrile (74: 26), wherein the sulfate buffer was prepared by dissolving 28.4 g anhydrous sodium sulfate in water, adding 2.7 ml phosphoric acid, adding 800 ml water, adjusting pH to 2.3 using ethanolamine, and adding water to the final volume of 1000 ml; flow rate: 1.0 mL/min; wavelength used in UV detection: 214 nm; column temperature: 40 °C; sample volume: 20 μL.

3. Samples:

Sample 1: Example 2

Sample 2: Example 1 in Chinese Application number 200510028990.0 in tht title of "Insulin nasal powder and preparation thereof". Specifically, it is a formulation containing 11.65% prescriptive insulin, 4.85% adhesive, 48.54% surfactant, 9.71 % cosurfactant, 8.74% lipid, 11.65% diluent, 4.85% acid/base. (The average diameter was 190 nm before lyophilization.)

Sample 3: insulin powder and a carrier mixture of mannitol and microcrystalline cellulose, the weight ratios are the same as in sample 1;

Dose: 2 mg insulin.

4. Results:

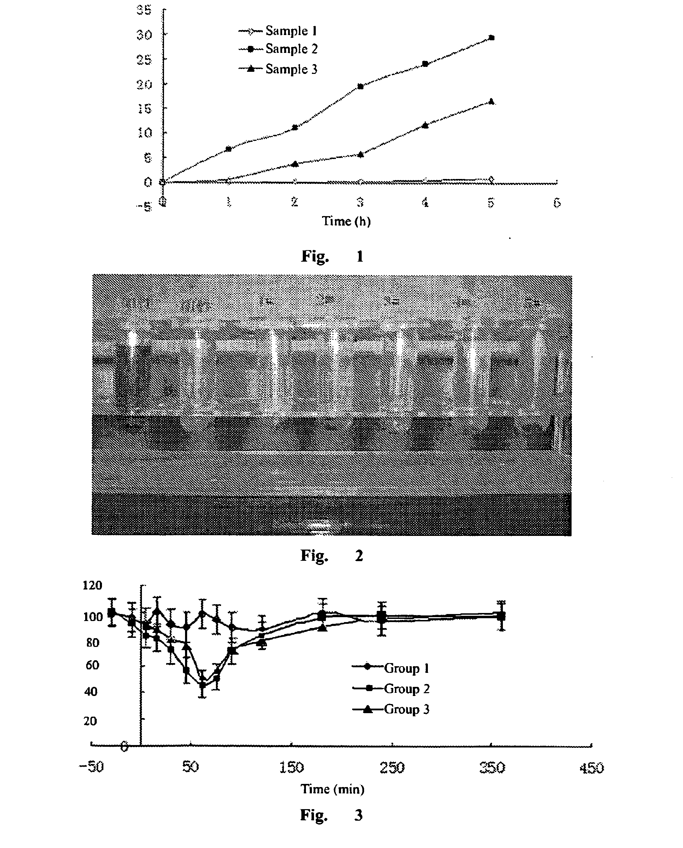

The results were shown in figure 1. It can be seen that the inhalable nasal powder preparation of insulin of the invention has a significantly higher capacity of transmucosal penetration than the conventional insulin powders.

The results suggest: the inhalable nasal powder preparation of insulin of the invention with the emulsifier and droplet diameter optimized, has the highest capacity of transmucosal penetration; the comparison with the insulin powder of sample 2 showed that the transmucosal penetration is significantly influenced by the ratio between the total surface area of hydrophobic cores of insulin to the total surface area of the oil droplets and the droplet size of nanoemulsion

Example 12

Irritation to Nasal Mucosa (*In Vivo*):

Evaluation of nasal mucosal ciliotoxicity:

Grouping (3 animals/group): Group 1: the inhalable nasal powder preparation of insulin of the invention (Example 5, regenerated in 1 mg/ml by normal saline); Group 2: saline; Group 3: 1 % propranolol. A toad was immobilized on a frog plate on its back. 0.2 ml of each of the above solutions was dripped into the palate mucosa of toads. After 0.5 h, a 3 mm × 3 mm specimen of the palate mucosa was removed and cleaned with saline. The specimen was spread on a glass slide and covered by a coverslip, and the movement of the mucosal cilia was observed under the microscopy. Then, the slide was placed in a sealed saturate tank which had been saturated with distilled water at room temperature (25 °C). The sample was removed for microscopy at points with a specified interval, and returned back to the saturate tank, until the motion of cilia could not be observed. The time of sustained motion of cilia was recorded as shown in table 1.

Results: In the group of saline, the cilia were clear, intact and active, most of the cilia was active, and the time of sustained motion was $678\pm26$ min. In the group of 1% propranolol, slight shedding of mucosal epithelium was observed, while the cilia were static. In the group of the inhalable nasal powder preparation of insulin of the invention, the cilia were fairly clear, and the time of sustained motion was $670\pm21$ min. The results indicate that the inhalable nasal powder preparation of insulin of the invention has a good compatibility with the nasal cilia, and does not inhibit the cilia motion.

Table 1. Time of Sustained Cilia motion

| Group | 1 | 2 | 3 |
|---|---|---|---|
| Time of motion (min) | $670\pm21$ | $678\pm26$ | 0 |
| Cilia Appearance | Clear and intact | Clear and intact | Shed, static |

Example 13

Irritation to Nasal Mucosa (*Ex Vivo*):

Evaluation of nasal mucosal ciliotoxicity:

Grouping (3 animals/group): Group 1: the inhalable nasal powder preparation of insulin of the invention (Example 10, regenerated in 1 mg/ml by normal saline); Group 2: saline; Group 3: 1 % propranolol. A toad was immobilized on a frog plate on its back. 3 mm × 3 mm specimens of the palate mucosa were obtained form toads and cleaned with saline. The specimen was spread on a glass slide, onto which each of the above solutions was dripped, and covered by a coverslip, and the movement of the mucosal cilia was observed under the microscopy. Afterwards, the slide was placed in a sealed saturate tank which had been saturated with distilled water at room temperature (25 °C). The sample was removed for microscopy at points with a given interval, and moved back to the saturate tank, until the motion of cilia could not be observed. The time of sustained motion of cilia was recorded, as shown in table 2.

Results: In the group of saline, the cilia were clear, intact and active, most of the cilia were active, and the time of sustained motion was $657\pm23$ min. In the group of 1 % propranolol, slight shedding of mucosal epithelium was observed, while the cilia were static. In the group of the inhalable nasal powder preparation of insulin of the invention, the cilia were fairly clear, and the time of sustained motion was $666\pm18$ min. The results indicate that the inhalable nasal powder preparation of insulin of the invention has a good compatibility with the nasal cilia and does not inhibit the cilia motion.

Table 2. Time of Sustained Cilia Motion

| Group | 1 | 2 | 3 |
|---|---|---|---|
| Time of motion (min) | $657\pm23$ | $666\pm18$ | 0 |
| Cilia Appearance | Clear and intact | Clear and intact | Shed, static |

Example 14

Hemolysis assay

One causes of nasal mucosal damage can be the destructive effect on cell membrane from some drugs and/or adjuvants. Thus, the nasal mucosal toxicity can be correlated to the drug's or adjuvant's effects on the biomembrane.

Preparation of a blood cell suspension: rabbit blood (about 20 ml) were added to a flask containing beads, and vortexed for 10 min or stirred by a glass rod to remove fibrinogen and obtain a sample of defibrinated blood. 10X volume of 0.9% sodium chloride solution was added and mixed to homogeneous, then centrifuged at 1200 r/min for 15 min, and the supernatant was removed. Then, 0.9% sodium chloride solution was added to the pellet, and the precipitated erythrocytes were washed by repeating the above procedure for additional three times, until the supernatant was no longer red. The obtained erythrocytes are formulated into a 2% suspension in 0.9% sodium chloride solution to be used in further experiments.

Preparation of the test samples:

The inhalable nasal powder preparation of insulin of the invention (Example 6) was dissolved in water to produce an isotonic solution. The solution is centrifuged and the supernatant was recovered and transferred into a separate container. Therein, the concentration of insulin was adjusted to I mg/ml using 0.9% sodium chloride solution.

Protocol: 7 clean tubes were numbered and marked from 1 to 7.Tubes 1 to 5 corresponded to test samples, tube 6 was a negative control, and tube 7 was a positive control. According to the amount and order specified in the table below, 2% erythrocyte suspension and 0.9% sodium chloride solution were added into the tubes, mixed well prior to incubation in an incubator at 37°C±0.5°C. They were observed every 15 min in the first hour, and then every 1 hour for the next 3 hours.

| Tube # | 1 | 2 | 3 | 4 | 5 | 6 | 7 |
|---|---|---|---|---|---|---|---|
| 2% Erythrocyte Suspension (ml) | 2.5 | 2.5 | 2.5 | 2.5 | 2.5 | 2.5 | 2.5 |
| Normal Saline (ml) | 2.0 | 2.1 | 2.2 | 2.3 | 2.4 | 2.5 | - |
| Distilled Water (ml) | - | - | - | - | - | - | 2.5 |
| Test Sample (ml) | 0.1 | 0.2 | 0.3 | 0.4 | 0.5 | - | - |

Results:

All the tubes were removed from the incubator after 3h, and centrifuged at 3000 r/m for 5 min. The results were shown in figure 2. In the positive control, the suspension was clear and showed a color of bright red, and no cell pellets were observed on the bottom of the tube, which indicated the occurrence of hemolysis. In the negative control, all the erythrocytes were pelleted, and the supernatant was clear and colorless, which indicated absence of hemolysis.

Discussion:

The fact that therein is no hemolysis observed in the negative control but in the positive control, while no hemolysis in the solutions containing test samples for the 3 hours of test indicates that the inhalable nasal powder preparation of insulin of the invention is well compatible with biomembranes.

Example 15

*In vivo* pharmacodynamic assay:

Animal and Administration:

12 2.5±0.2 kg weighted rabbits (6 females and 6 males)were divided into two groups randomly. Group 1: insulin powder, Group 2: the inhalable nasal powder preparation of insulin prepared as described in Example 8 of the invention; Group 3: the regenerated solution of the inhalable nasal powder preparation of insulin prepared as described in Example 8 of the invention. The experiment was stated after the animals were conditioned for 7 days. The animals were fasted from the night immediately before the experiment, but let free to water.

Dosage of administration: 25 IU/animal

Blood samples were collected from ear veins at the time points of 30 and 10 min before the administration, and 0, 5, 15, 30, 45, 60, 75, 90, 120, 180, 240, 300, and 360 min after the administration. The serum level of glucose was determined by the glucose oxidase assay, and the results were shown in figure 3.

It can be seen from figure 3 that after nasal administration, the inhalable nasal powder preparation of insulin of the invention effectively facilitated the entry of insulin into the circulation through the nasal mucosa, and showed a significant hypoglycemic effect. The regenerated solution showed no significant different in hypoglycemic effect. These indicated that the inhalable powder of insulin can be absorbed rapidly via nasal administration. By contrary, no hypoglycemic effect was observed with the insulin powder, which indicated that the dry insulin powder could not be effectively absorbed via nasal administration.

Example 16

*In vivo* pharmacodynamic assay:

Animal and Administration:

18 2.5±-0.2 kg weighted rabbits (6 females and 6 males)were divided into 3 groups randomly. The experiment was stated after the animal were conditioned for 7 days. The animals were fasted from the night immediately before the experiment, but let free to water. To group 1 was administered the inhalable nasal powder preparation of insulin prepared as described in Example 1; to group 2 was administered the inhalable nasal powder preparation of insulin prepared as described in Example 2; and to group 3 was administered the inhalable nasal powder preparation of insulin prepared as described in Example 3 .

Dosage of administration: 25 IU/animal

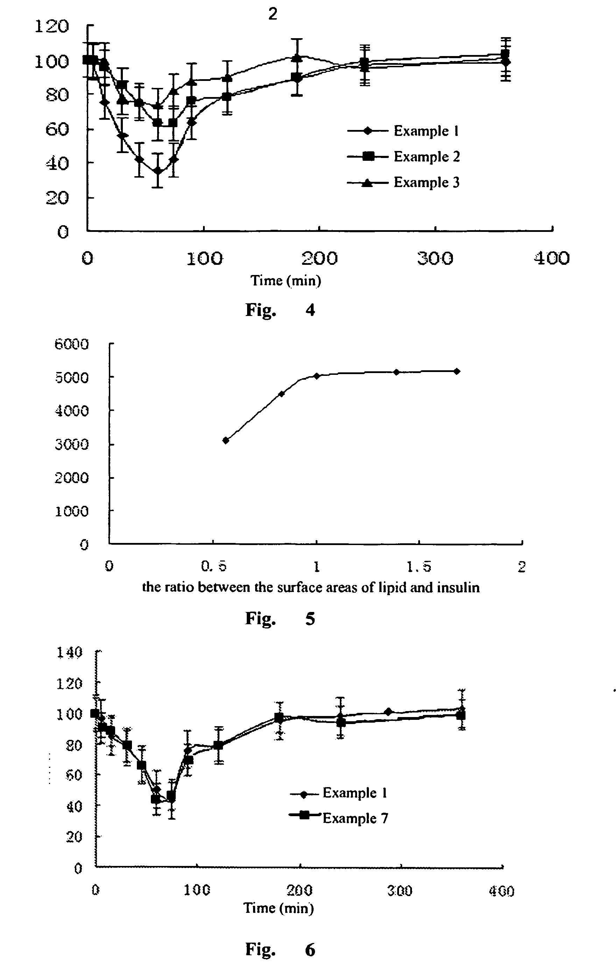

Blood samples were collected from ear veins of rabbits before administration and at the time points of 5, 15, 30, 45, 60, 75, 90, 120, 180, 240, and 360 min after the administration. The serum level of glucose was determined by the glucose oxidase assay to obtain the average blood glucose level, and the results were shown in figure 4.

It can be seen that, with the same prescription, the droplets in the insulin microemulsion before lyophilization have a smaller diameter and a larger surface area, and a significant hypoglycemic effect can be observed at a ratio between the surface areas of insulin to oil droplets in the range of 1:1~1.5, and the effect decreases below minimum. The results support that the ratio between the surface areas of insulin and oil droplets is relevant to the hypoglycemic effect.

Example 17

*In vivo* pharmacodynamic assay:

Preparation of sample suspensions:

The blank microemulsion was prepared as described in Example 4. The inhalable powders of insulin were prepared by mixing insulin with the blank microemulsion at the ratios of 1:10, 1:15, 1:18, 1:25, and 1:30 (g/ml), and the remaining components and amounts are the same among the samples.

Animal and Administration:

15 2.5±0.2 kg weighted rabbits (7 females and 8 males) were divided into 5 groups randomly. The experiment was stated after the animal were conditioned for 7 days. The animals were fasted from the night immediately before the experiment, but let free to water. The above insulin formulations were administered to those groups, respectively.

Dosage of administration: 25 IU/animal

Blood samples were collected from ear veins of rabbits before administration and at the time points of 5, 15, 30, 45, 60, 75, 90, 120, 180, 240, and 360 min after the administration. The serum level of glucose was determined by the glucose oxidase assay to obtain the average blood glucose level. The comparison of the areas above blood glucose curves of all the formulations (wherein the diameters thereof are approximate to each other but the ratios of lipid to insulin are different) was shown in figure 5.

It can be seen from results that, when the particle diameters were the same (about 60 nm), the area over the curve of blood glucose remained substantially unchanged when the ratio of insulin to blank emulsion is kept no less than than 1:18 (g/ml), corresponding to a ratio of insulin to lipid of no less than 1:0.77 (corresponding to a ratio of hydrophobic areas between the oil droplets and the insulin being 1.0). And decreased with the ratio going down and ratio of insulin to lipid going up.

Example 18

*In vivo* pharmacodynamic assay:

Animal and Administration:

6 10.0±0.5 kg weighted Beagle dogs (3 females and 3 males) were divided into two groups randomly. The experiment was stated after the animal were conditioned for 7 days. The animals were fasted from the night immediately before the experiment, but let free to water. To group 1 was administered the inhalable nasal powder preparation of insulin prepared as described in Example 1, and to group 2 was administered the inhalable nasal powder preparation of insulin prepared as described in Example 7.

Dosage of administration: 3 IU/kg

Blood samples were collected from ear veins of rabbits before administration and at the time points of 5, 15, 30, 45, 60, 75, 90, 120, 180, 240, and 360 min after the administration. The serum level of glucose was determined by the glucose oxidase assay to obtain the average blood glucose level, and the results were shown in figure 6.
It can be seen from results that both of the formulations can substantially reduce the blood glucose level, difference being insignificant. The ratios of insulin to lipid are 1:0.67 and 1:1 (g/ml) for examples 1 and 7 respectively, the diameters of droplets are 45 and 60 nm r, and the ratios of hydrophobic areas between the oil droplets and the insulin are 1.15:1 and 1.27:1. The result is consistent with the above that the a ratio insulin and lipid meet the range of surface area ratio being 1:1 to 1.5:1, gives equivalently good hypoglycemic effects.

Example 19

Pharmacokinetic assay

Methods: The exogenous insulin was detected by the Control day method. The animals' endogenous insulin level and C peptide level were measured one week before the experiment, and the F factor at different time points were calculated. The concentration of the endogenous insulin can be calculated from the concentration of C peptide in the blood sample, and then, the concentration of the exogenous insulin at each time point can be calculated from the concentration of total insulin in blood as detected. This was used to evaluate the difference in pharmacokinetics given by the different routes of administration. And, the safety of medication were evaluated by observing the vital measurements at set time points.
Animal and protocols: That was an open, random, and crossover study with an interval between experiments of 1 week. 6 Beagle dogs (3 females and 3 males) with a body weight of 8-10 kg were used in the experiment. The animals were divided into 2 groups (3 animals/group). The week, the blood glucose level, endogenous insulin, and C peptide were determined as the blank controls. The second week, animals of group A were subcutaneously administered with an insulin injection, and group B were nasally administered with the nasal inhalable powder of insulin. The next week, the administration is crossed by giving each animal an injection of human insulin (Novolin®R) (6 IU) or an aliquot of nasal inhalable powder of recombinant insulin (25 IU) on different days. The fourth week, the animals were given an insulin spray (25 IU). Blood samples were collected before and after each administration.

Drugs:

Insulin injection (Novolin®R)
Inhalable powder of insulin, as described in example 9 (25 IU/animal)
Insulin spray, the formulation before lyophilization in example 9 (25 IU/animal)

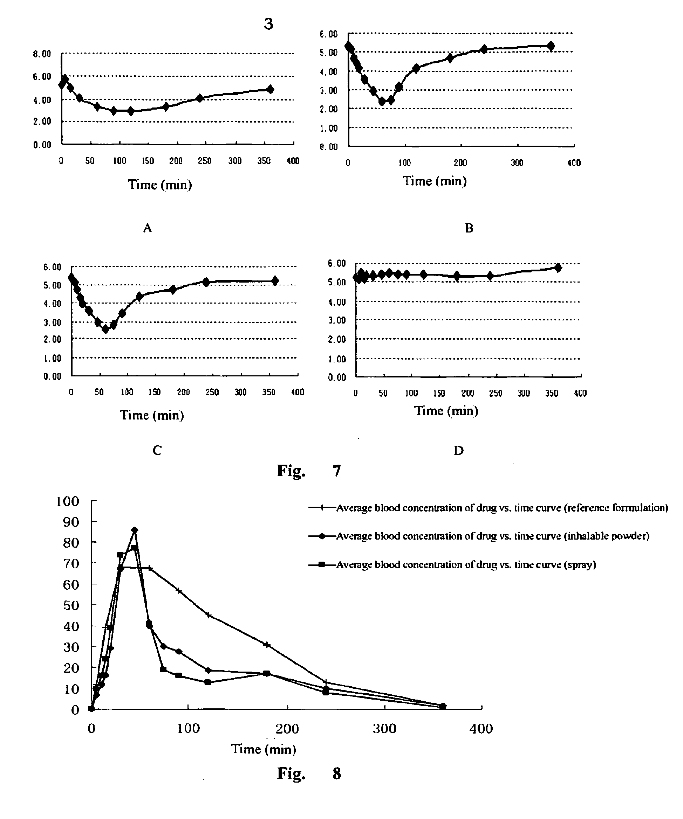

Protocol: The animals were fasted the night before the experiment. The Beagle dogs were anesthetized using 3% pentobarbital on the next morning, and then administered with the reference formulations or the test formulations. For the group receiving subcutaneous administration, 3-4 ml of blood samples were collected from the rear leg before administration and 5, 15, 30, 60, 90, 120, 180, 240, and 360 minutes after administration. For the group receiving nasal administration, 3-4 ml of blood samples were collected from the rear leg before administration and 5, 10, 15, 20, 30, 45, 60, 75, 90, 120, 180, 240, and 360 minutes after administration. 60 $\mu$l of each collected samples was used to determine the glucose concentration, which was plotted as the average blood glucose level curve as shown in figure 7. Figure 7A showed the average blood glucose level curve obtained with subcutaneous injection of insulin, Figure 7B with nasal administration of inhalable powder of insulin, Figure 7C with nasal administration of insulin spray and Figure 7D with the blank control. The rest of each sample was separated to obtain the serum, and measured for the blood levels of the drug and the C peptide, then, the blood concentration of exogenous insulin were calculated and plotted against time as shown in figure 8.

Data Analysis:

The measured blood glucose level data were processed by the DAS software to calculate the area over the curve (blood glucose lever vs. time) using the trapezoidal method. The values of relative bioavailability were calculated to be 15.1 % for the inhalable powder group and 14.0% for the spray. The measured blood levels of the drug were processed by the DAS software to calculate the area under the curve (the blood concentration of drug vs. time). As compared with subcutaneous injection of insulin, the bioavailability is 15.41% for the inhalable powder, and 13.59% for the spray, which indicated that the inhalable powder provided a fairly higher drug absorption than the spray.

Example 20

Topical irritation on mucosa

Animal and grouping: 15 2.19$\pm$0.16 kg weighted rabbits (8 females and 7 males)are divided into 3 groups (5 rabbits/group):
A: Normal: no drug or any other treatment.
B: Nasal inhalable powder of insulin (Example 10).
C: Excipient of in the form of an inhalable powder.
Dosing: for each animal, one capsule ( 1 mg insulin) per day for 7 days.

Measurements and agenda: The rabbits were observed for response to irritation for the 30 minutes after administration every day. The rabbit was sacrificed and autopsy 24 hrs after the final administration (i.e., day 8). The nasal cavity was examined for the conditions including hemorrhagic spot, congestion and inflammation on the musocal surface in the drug group and the excipient group. The specimen of nasal mucosa was fixed in 10% formalin for 5-7 days, embedded in paraffin and cut into 5 $\mu$m pieces, then dewaxed and subject to a gradient dehydration with ethanol, and stained by HE for microscopic observation and photography

Results:

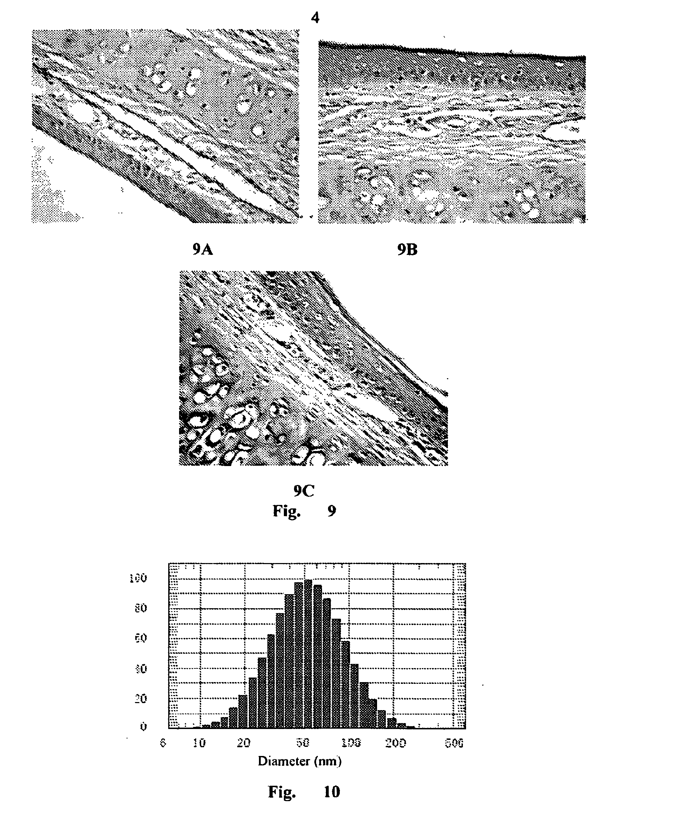

Anomalies in such as sneeze, nose scratching, congestion, edema, secretion were not observed after administration. And, the autopsy showed no hemorrhagic spot, congestion or edema in the drug group and the excipient group. No infiltration of inflammatory cells or abnormal hyperplasia/atrophy was observed in the pathological section of the nasal mucosae, as shown in figure 9 (A: no-treatment control group, B: drug administration group, C: excipient group; $\times$400, stained by HE). No nasal irritation was observed after nasal administration of inhalable powder of insulin, which indicated an excellent safety of medication.

Example 21

Measurement of the droplet size in the blank microemulsion and the insulin nanoemulsion, and Measurement of the zetapotential.
The blank microemulsion was prepared according to Example 9, and an emulsion with the ratio (w/v) of insulin to lipid of 1:0.8 (g/ml) were prepared. The droplet diameter and the zeta-potential in both were measured, and results shown in figures 10-13.
It can be seen that the droplet size increased with addition of insulin into the microemulsion, which indicated that interaction occurred between the two, which was further confirmed by the change in zeta potential as measured.

Example 22

Repose angle of the inhalable powder

The inhalable powder of insulin of each example was allowed to freefall from the funnel of the Repose angle measurement device to form a bulk of powder on a disc. The bulk radius (r) and height (h) were measured to calculate the repose angle ($\theta$) according to the equation of $\theta$=-h/r, giving the results as shown below.

| Example | 1 | 2 | 3 | 4 | 5 |
|---|---|---|---|---|---|
| Repose Angle | 40.5 | 41.2 | 42.8 | 42.2 | 38.7 |
| Example | 6 | 7 | 8 | 9 | 10 |
| Repose Angle | 40.0 | 41.2 | 39.6 | 40.5 | 41.6 |

Example 23

Cytotoxicity Assay:

Methods: L929 cells (mouse fibroblast cell) were passaged for 72 h, and digested into a cell suspension. 1 ml of the cell suspension was added to each well of a 24-well plate, and the plate was incubated in an incubator for 24 h at 37°C with 5% $CO_2$. Thereafter, the medium were sucked out and replaced with a 50% drug solution for the test group and a 50% positive control solution for the positive control group. The negative control group were replaced and incubated with fresh medium. The plates were put back into the incubator for continued incubation. 2, 4 and 7 days later, the cells were observed and counted under a microscope, and the value of relative growth rate (RGR) of the cells was calculated to evaluate the cytotoxicity.

Preparation of the samples:

1) The inhalable powder of insulin of Example 9 was regenerated in saline, the supernatant after centrifugation was recovered and diluted to a insulin concentration of 0.8 mg/ml.
2) Blank powder was prepared using the blank emulsion (without insulin) as Example 9, and regenerated and formulated as above to obtain a blank solution without insulin.
3) USP positive control: The materials were washed and autoclaved (121°C, 15 min), then mixed into the medium at a ratio of 6 $cm^2$/ml, and the mixture was incubated for 24 h at 37°C to obtain the suspension.

Evaluation and Scores: Relative Growth Rate of the Cells.

| Score of Response | Relative Growth Rate (RGR) |
|---|---|
| 0 | >=100 |
| 1 | 75-99 |
| 2 | 50-74 |
| 3 | 25-49 |
| 4 | 1-24 |
| 5 | 0 |

The results were shown in the table below. It can be seen that the regenerated solution prepared with the inhalable powder of insulin of the invention did not show any toxicity to the cells, which indicated an excellent safety of medication.

| Time | Group | Cell Count ($\times 10^4$) | RGR | Score |
|---|---|---|---|---|
| Day 2 | 1 | 2.33±0.38 | 84.85 | 1 |
| | 2 | 2.58±0.29 | 93.94 | 1 |
| | 3 Positive | 0.00±0.00 | 0 | 5 |
| | Negative | 2.75±0.25 | | |
| Day 4 | 1 | 12.75±1.00 | 88.95 | 1 |
| | 2 | 13.17±1.38 | 91.86 | 1 |
| | 3 Positive | 0.00±0.00 | 0 | 5 |
| | Negative | 14.33±0.63 | | |
| Day 7 | 1 | 71.08±2.79 | 93.74 | 1 |
| | 2 | 71.58±2.92 | 94.40 | 1 |
| | 3 Positive | 0.00±0.00 | 0 | 5 |
| | Negative | 75.83±1.63 | | |

## Claims

1. A self-microemulsifiable powder of an insulin formulation, comprising insulin, lipid, emulsifier, antioxidant, supporting agent, bio-gel adhesive, and pH modifier; wherein, for each gram of insulin, the formulation comprises:

   | | |
   |---|---|
   | antioxidant | 0.005 ~ 0.045 g, |
   | supporting agent | 0 ~ 5 g, |
   | bio-gel adhesive | 0.01 ~ 1 g, and |
   | pH modifier | 0.01 ~ 1.0 g; |

   wherein
   the amount of lipid meets the following equations:

   the total surface area of the hydrophobic cores of insulin : the total surface area of oil droplet = 1 : 0.5~2, wherein the average diameter of the oil droplets is about 20 to 200 nm, and
   the ratio (w/v) of lipid : emulsifier = 1 ml : 0.6~1.2 g;
   the said emulsifier comprises lecithin and Tween 80, and wherein
   the supporting agent is selected from the group consisting of lactose, mannitol, xylitol, sorbitol and their combinations, and
   the bio-gel adhesive is selected from the group consisting of chitosan, alginate, gum arabic, hydroxypropyl methylcellulose, hydroxypropyl cellulose, sodium carboxymethylcellulose, and their combinations.

2. The self-microemulsifiable powder of an insulin formulation of claim 1, wherein, for each gram of insulin, the formulation comprises:

   | | |
   |---|---|
   | antioxidant | 0.015 ~ 0.03 g, |
   | supporting agent | 1.0 ~ 2.0 g, |
   | bio-gel adhesive | 0.1 ~ 0.5 g, and |
   | pH modifier | 0.02 ~ 0.5 g; and |

   wherein the total surface area of the hydrophobic cores of insulin: the total surface area of oil droplet = 1 : 1~ 1.5.

3. The self-microemulsifiable powder of an insulin formulation of claim 1, wherein, for each gram of insulin, the powder comprises:

| antioxidant | 0.005 ~ 0.045 g, |
|---|---|
| supporting agent | 0 ~ 5 g, |
| bio-gel adhesive | 0.01 ~ 1 g, |
| pH modifier | 0.01 ~ 1.0 g, |
| lipid | 0.13 ~ 2.6 ml, |
| emulsifier | 0.24 ~ 3.0 g; |

wherein the average diameter of oil droplets is about 40 to 100 nm.

4. The self-microemulsifiable powder of an insulin formulation of claim 3, wherein, for each gram of insulin, the powder comprises:

| antioxidant | 0.015 ~ 0.03 g, |
|---|---|
| supporting agent | 1.0 ~ 2.0 g, |
| bio-gel adhesive | 0.1 ~ 0.5 g, |
| pH modifier | 0.02 ~ 0.5 g, |
| lipid | 0.51 ~ 1.3 ml, and |
| emulsifier | 0.4 ~ 1.4 g. |

5. The self-microemulsifiable powder of an insulin formulation of claim 1, wherein the ratio (w/w) of lecithin : Tween 80 is 1 : 0.10~0.4.

6. The self-microemulsifiable powder of an insulin formulation of claim 1, wherein the lipid is selected from the group consisting of a synthetic lipid, a natural lipid and their combination.

7. The self-microemulsifiable powder of an insulin formulation of claim 6, wherein the lipid is selected from the group consisting of soy oil, tea oil, decanoyl and octanoyl glycerides, and their combinations.

8. The self microemulsifiable powder of an insulin formulation of claim 1, wherein the pH modifier is selected from the group consisting of HCl, NaOH, an organic acid, an organic base, and their combinations.

9. The self-microemulsifiable powder of an insulin formulation of claim 1, wherein the antioxidant is selected from the group consisting of ascorbyl palmitate, t-butyl-p-hydroxyanisole, propyl gallate, vitamin E, and their combinations.

10. The self microemulsifiable powder of an insulin formulation of claim 1, wherein the average diameter of the powder is about 10 ~ 150 $\mu$m.

11. A nanoemulsion of insulin used for preparing a self-microemulsifiable powdered formulation of insulin according to any one of claims 1-10, additionally comprising, for each gram of insulin, about 6 to 96 ml of water.

12. An inhalable nasal powder preparation of insulin, comprising a self microemulsifiable powdered formulation of insulin according to any one of claims 1-11 and a pharmaceutically acceptable carrier.

13. A method for preparing an inhalable nasal powder preparation of insulin according to claim 12, comprising:

preparation of the nanoemulsion of insulin, comprising:

(1) dissolving bio-gel adhesive in water or a HCl solution to produce a mixture of the bio-gel adhesive and water or the HCl solution;
(2) mixing the lipid, surfactant, and antioxidant to dissolve, mixing the obtained oil phase with water and stirring into a primary emulsion, homogenizing the obtained primary emulsion in an emulsion homogenizer for 2 ~ 10 cycles under a pressure between about 400 - 800 bars to produce a semitransparent or transparent microemulsion, wherein the average diameter of oil droplets is about 10 to 200 nm;
(3) mixing a specified amount of insulin with the microemulsion obtained in step (2) and the hydrogel containing mixture of step (1), and adding 1-6 mol/L HCl or other acids to dissolve insulin;
(4) adding the supporting agent, and adjusting the pH to 3.5 ~ 8.5 with the pH modifier, to obtain a nanoemulsion of insulin;

preparation of the self-microemulsifiable powder of insulin, comprising:

subjecting the nanoemulsion of insulin obtained above to lyophilizing or spray-drying, and sieving for the particles having a size of about 10 to 150 μm as the self microemulsifiable powder of insulin;
preparation of an inhalable nasal powder preparation of insulin:

mixing the self microemulsifiable powder of insulin obtained above with a carrier, to obtain an inhalable nasal powder preparation of insulin,
wherein
the supporting agent is selected from the group consisting of lactose, mannitol, xylitol, sorbitol and their combinations, and
the bio-gel adhesive is selected from the group consisting of chitosan, alginate, gum arabic, hydroxypropyl methylcellulose, hydroxypropyl cellulose, sodium carboxymethylcellulose, and their combinations.

## Patentansprüche

1. Ein selbstmikroemulgierbares Pulver einer Insulinformulierung, das Insulin, Lipid, Emulgiermittel, Antioxidans, Trägerstoff, Bio-Gelklebstoff und pH-Modifikator umfasst, wobei die Formulierung pro Gramm Insulin umfasst:

| Antioxidans | 0,005 ~ 0,045 g, |
|---|---|
| Trägerstoff | 0 ~ 5 g, |
| Bio-Gelklebstoff | 0,01 ~ 1 g, und |
| pH-Modifikator | 0,01 ~ 1,0 g; |

wobei
die Menge an Lipid folgende Gleichungen erfüllt:

die gesamte Oberfläche der hydrophoben Kerne des Insulins : der gesamten Oberfläche eines Öltropfens = 1 : 0,5 ~ 2, wobei der mittlere Durchmesser der Öltropfen etwa 20 bis 200 nm beträgt, und
das Verhältnis (w/v) von Lipid : Emulgiermittel = 1 ml : 0,6 ~ 1,2 g;
das Emulgiermittel Lecithin und Tween 80 umfasst, und wobei der Trägerstoff ausgewählt ist aus der Gruppe bestehend aus Laktose, Mannit, Xylit, Sorbit und deren Kombinationen, und
der Bio-Gelklebstoff ausgewählt ist aus der Gruppe bestehend aus Chitosan, Alginat, Gummiarabikum, Hydroxypropylmethylcellulose, Hydroxypropylcellulose, Natrium-carboxymethylcellulose und deren Kombinationen.

2. Das selbstmikroemulgierbare Pulver einer Insulinformulierung gemäß Anspruch 1, wobei die Formulierung pro Gramm Insulin umfasst:

| Antioxidans | 0,015 ~ 0,03 g, |
|---|---|
| Trägerstoff | 1,0 ~ 2,0 g, |
| Bio-Gelklebstoff | 0,1 ~ 0.5 g, und |
| pH-Modifikator | 0,02 ~ 0,5 g; und |

wobei die gesamte Oberfläche der hydrophoben Kerne des Insulins : der gesamten Oberfläche eines Öltropfens = 1 : 1 ~ 1,5.

3. Das selbstmikroemulgierbare Pulver einer Insulinformulierung gemäß Anspruch 1, wobei die Formulierung pro Gramm Insulin umfasst:

| Antioxidans | 0,005 ~ 0,045 g, |
|---|---|

(fortgesetzt)

| | |
|---|---|
| Trägerstoff | 0 ~ 5 g, |
| Bio-Gelklebstoff | 0,01 ~ 1 g, |
| pH-Modifikator | 0,01 ~ 1,0 g; |
| Lipid | 0,13 ~ 2,6 ml, |
| Emulgiermittel | 0,24 ~ 3,0 g; |

wobei der mittlere Durchmesser der Öltropfen etwa 40 bis 100 nm beträgt.

4. Das selbstmikroemulgierbare Pulver einer Insulinformulierung gemäß Anspruch 3, wobei die Formulierung pro Gramm Insulin umfasst:

| | |
|---|---|
| Antioxidans | 0,015 ~ 0,03 g, |
| Trägerstoff | 1,0 ~ 2,0 g, |
| Bio-Gelklebstoff | 0, 1 ~ 0,5 g, |
| pH-Modifikator | 0,02 ~ 0,5 g; |
| Lipid | 0,51 ~ 1,3 ml, und |
| Emulgiermittel | 0,4 ~ 1,4 g. |

5. Das selbstmikroemulgierbare Pulver einer Insulinformulierung gemäß Anspruch 1, wobei das Verhältnis (w/w) von Lecithin : Tween 80 1: 0,10 ~ 0,4 beträgt.

6. Das selbstmikroemulgierbare Pulver einer Insulinformulierung gemäß Anspruch 1, wobei das Lipid ausgewählt ist aus der Gruppe bestehend aus einem synthetischen Lipid, einem natürlichen Lipid und deren Kombination.

7. Das selbstmikroemulgierbare Pulver einer Insulinformulierung gemäß Anspruch 6, wobei das Lipid ausgewählt ist aus der Gruppe bestehend aus Sojaöl, Öl aus Tee, Decanoyl- und Octanoylglyceriden und deren Kombinationen.

8. Das selbstmikroemulgierbare Pulver einer Insulinformulierung gemäß Anspruch 1, wobei der pH-Modifikator ausgewählt ist aus der Gruppe bestehend aus HCl, NaOH, einer organischen Säure, einer organischen Base und deren Kombinationen.

9. Das selbstmikroemulgierbare Pulver einer Insulinformulierung gemäß Anspruch 1, wobei das Antioxidans ausgewählt ist aus der Gruppe bestehend aus Ascorbylpalmitat, t-Butyl-p-hydroxyanisol, Propylgallat, Vitamin E und deren Kombinationen.

10. Das selbstmikroemulgierbare Pulver einer Insulinformulierung gemäß Anspruch 1, wobei der mittlere Durchmesser des Pulvers etwa 10 ~ 150 $\mu$m beträgt.

11. Eine Insulinnanoemulsion, verwendet zur Herstellung einer selbstmikroemulgierbaren pulverförmigen Formulierung von Insulin gemäß einem der Ansprüche 1 bis 10, die zusätzlich etwa 6 bis 96 ml Wasser pro Gramm Insulin umfasst.

12. Eine inhalierbare Nasenpulverzubereitung von Insulin, die eine selbstmikroemulgierbare pulverförmige Formulierung von Insulin gemäß einem der Ansprüche 1 bis 11 und einen pharmazeutisch verträglichen Träger umfasst.

13. Ein Verfahren zur Herstellung einer inhalierbaren Nasenpulverzubereitung von Insulin gemäß Anspruch 12, umfassend:

Herstellung der Nanoemulsion von Insulin, umfassend:

(1) Lösen eines Bio-Gelklebstoffs in Wasser oder einer HCl-Lösung, um ein Gemisch des Bio-Gelklebstoffs und Wasser oder der HCl-Lösung herzustellen;
(2) Mischen des Lipids, des oberflächenaktiven Mittels und des Antioxidans, um diese zu lösen, Mischen der erhaltenen Ölphase mit Wasser und Rühren zu einer ersten Emulsion, Homogenisieren der erhaltenen ersten Emulsion in einem Emulsionshomogenisator in 2 - 10 Zyklen unter einem Druck zwischen etwa 400 bis 800 bar, um eine halbtransparente oder transparente Microemulsion herzustellen, wobei der mittlere Durchmesser der Öltropfen etwa 10 bis 200 nm beträgt;

(3) Mischen einer bestimmten Menge an Insulin mit der in Schritt (2) erhaltenen Microemulsion und dem Hydrogel enthaltenden Gemisch aus Schritt (1), und Zugeben von 1 bis 6 mol/l HCl oder anderer Säuren, um das Insulin zu lösen;

(4) Zugeben des Trägermittels und Einstellen des pH-Werts auf 3,5~- 8,5 mit dem pH-Modifikator, um eine Insulin-Nanoemulsion zu erhalten;

Herstellung des selbstmikroemulgierbaren Pulvers von Insulin, umfassend:

Unterziehen der oben erhaltenen Insulin-Nanoemulsion einer Gefriertrocknung oder Sprühtrocknung und Absieben der Teilchen mit einer Größe von etwa 10 bis 150 $\mu$m als das selbstmikroemulgierbare Pulver von Insulin;

Herstellung der inhalierbaren Nasenpulverzubereitung von Insulin:

Mischen des oben erhaltenen selbstmikroemulgierbaren Pulvers von Insulin mit einem Träger, um eine inhalierbare Nasenpulverzubereitung von Insulin zu erhalten, wobei

der Trägerstoff ausgewählt ist aus der Gruppe bestehend aus Laktose, Mannit, Xylit, Sorbit und deren Kombinationen, und

der Bio-Gelklebstoff ausgewählt ist aus der Gruppe bestehend aus Chitosan, Alginat, Gummiarabikum, Hydroxypropylmethylcellulose, Hydroxypropyl-cellulose, Natriumcarboxymethylcellulose und deren Kombinationen.

**Revendications**

1. Poudre auto-microémulsifiable de formulation d'insuline, comprenant de l'insuline, un lipide, un émulsifiant, un antioxydant, un agent de support, un adhésif bio-gel et un agent modifiant le pH ; dans laquelle, pour chaque gramme d'insuline, la formulation comprend :

| | |
|---|---|
| antioxydant | 0,005 ~ 0,045 g |
| agent de support | 0 ~ 5 g, |
| adhésif bio-gel | 0,01 ~ 1 g et |
| modificateur du pH | 0,01 ~ 1,0 g ; |

dans laquelle
la quantité de lipide répond aux équations suivantes :

la surface totale des noyaux hydrophobes de l'insuline : la surface totale d'une gouttelette d'huile = 1 : 0,5 ~ 2, dans laquelle le diamètre moyen des gouttelettes d'huile est d'environ 20 à 200 nm, et
le rapport (p/v) du lipide : émulsifiant = 1 ml : 0, 6 ~ 1,2 g ;
ledit émulsifiant comprenant de la lécithine et du Tween 80, et dans laquelle
l'agent de support est choisi dans le groupe comprenant le lactose, le mannitol, le xylitol, le sorbitol et leurs combinaisons, et
l'adhésif bio-gel est choisi dans le groupe comprenant le chitosan, l'alginate, la gomme arabique, l'hydroxypropylméthyl-cellulose, l'hydroxypropylpropyl-cellulose, la carboxyméthylcellulose sodium et leurs combinaisons.

2. Poudre auto-microémulsifiable de formulation d'insuline selon la revendication 1, dans laquelle, pour chaque gramme d'insuline, la formulation comprend :

| | |
|---|---|
| antioxydant | 0,015 ~ 0,03 g, |
| agent de support | 1,0 ~ 2, 0 g, |
| adhésif bio-gel | 0,1 ~ 0,5 g et |

(suite)

| | |
|---|---|
| modificateur du pH | 0,02 ~ 0,5 g ; et |

dans laquelle la surface totale des noyaux hydrophobes de l'insuline : la surface totale de gouttelette d'huile = 1 : 1 ~ 1,5.

3. Poudre auto-microémulsifiable de formulation d'insuline selon la revendication 1, dans laquelle, pour chaque gramme d'insuline, la formulation comprend :

| | |
|---|---|
| antioxydant | 0,005 ~ 0,045 g, |
| agent de support | 0 ~ 5 g, |
| adhésif bio-gel | 0,01 ~ 1 g |
| modificateur du pH | 0,01 ~ 1,0 g |
| lipide | 0,13 ~ 2,6 ml |
| émulsifiant | 0,24 ~ 3,0 g ; |

dans laquelle le diamètre moyen des gouttelettes d'huile est d'environ 40 à 100 nm.

4. Poudre auto-microémulsifiable de formulation d'insuline selon la revendication 3, dans laquelle, pour chaque gramme d'insuline, la formulation comprend :

| | |
|---|---|
| antioxydant | 0,015 ~ 0,03 g |
| agent de support | 1,0 ~ 2,0 g, |
| adhésif bio-gel | 0,1 ~ 0,5 g |
| modificateur du pH | 0,02 ~ 0,5 g |
| lipide | 0,51 ~ 1,3 ml et |
| émulsifiant | 0,4 ~ 1,4 g. |

5. Poudre auto-microémulsifiable de formulation d'insuline selon la revendication 1, dans laquelle le rapport (p/p) de lécithine : Tween 80 est de 1 : 0,10 - 0,4.

6. Poudre auto-microémulsifiable de formulation d'insuline selon la revendication 1, dans laquelle le lipide est choisi dans le groupe comprenant un lipide synthétique, un lipide naturel et leur combinaison.

7. Poudre auto-microémulsifiable de formulation d'insuline selon la revendication 6, dans laquelle le lipide est choisi dans le groupe comprenant l'huile de soja, l'huile de thé, les glycérides décanoyle et octanoyle et leurs combinaisons.

8. Poudre auto-microémulsifiable de formulation d'insuline selon la revendication 1, dans laquelle le modificateur du pH est choisi dans le groupe comprenant le HCl, le NaOH, un acide organique, une base organique et leurs combinaisons.

9. Poudre auto-microémulsifiable de formulation d'insuline selon la revendication 1, dans laquelle l'antioxydant est choisi dans le groupe comprenant le palmitate d'ascorbyle, le t-butyl-p-hydroxyanisole, le propyl-gallate, la vitamine E et leurs combinaisons.

10. Poudre auto-microémulsifiable de formulation d'insuline selon la revendication 1, dans laquelle le diamètre moyen de la poudre est d'environ 10 - 150 $\mu$m.

11. Nano-émulsion d'insuline utilisée pour préparer une formulation en poudre auto-microémulsifiable d'insuline selon l'une quelconque des revendications 1 à 10, comprenant en outre, pour chaque gramme d'insuline, environ 6 à 96 ml d'eau.

12. Préparation de poudre nasale inhalable d'insuline, comprenant une formulation en poudre auto-microémulsifiable d'insuline selon l'une quelconque des revendications 1 à 11 et un vecteur pharmaceutiquement acceptable.

**13.** Procédé de préparation d'une préparation en poudre nasale inhalable d'insuline selon la revendication 12, comprenant :

la préparation de la nano-émulsion d'insuline comprenant :

(1) la dissolution de l'adhésif bio-gel dans de l'eau ou une solution de HCl pour produire un mélange d'adhésif bio-gel et d'eau ou de solution de HCl ;

(2) le mélange de lipide, de tensioactif et d'antioxydant jusqu'à dissolution, le mélange de la phase huileuse obtenue avec de l'eau et l'agitation en une émulsion primaire, l'homogénéisation de l'émulsion primaire obtenue dans un homogénéisateur d'émulsion pendant 2 - 10 cycles sous une pression entre 400 et 800 bars pour produire une microémulsion semi-transparente ou transparente, dans laquelle le diamètre moyen des gouttelettes d'huile est d'environ 10 à 200 nm ;

(3) le mélange d'une quantité spécifiée d'insuline avec la microémulsion obtenue à l'étape (2) et le mélange contenant de l'hydrogel de l'étape (1), et l'addition de 1 à 6 moles/L de HCl ou d'autres acides pour dissoudre l'insuline ;

(4) l'addition de l'agent de support et l'ajustement du pH à 3,5 - 8,5 avec le modificateur du pH, pour obtenir une nano-émulsion d'insuline ;

la préparation de la poudre auto-microémulsifiable d'insuline, comprenant :

la soumission de la nano-émulsion d'insuline obtenue ci-dessus à une lyophilisation ou un séchage par pulvérisation et le tamisage des particules ayant une taille d'environ 10 à 150 $\mu$m en tant que poudre auto-microémulsionable d'insuline ;

la préparation d'une préparation en poudre nasale inhalable d'insuline :

le mélange de la poudre auto-microémulsifiable d'insuline obtenue ci-dessus avec un support pour obtenir une préparation de poudre nasale inhalable d'insuline, dans lequel

l'agent de support est choisi dans le groupe comprenant le lactose, le mannitol, le xylitol, le sorbitol et leurs combinaisons, et

l'adhésif bio-gel est choisi dans le groupe comprenant le chitosan, l'alginate, la gomme arabique, l'hydroxypropylméthyl-cellulose, l'hydroxypropyl-cellulose, la carboxyméthylcellulose de sodium et leurs combinaisons.

**Fig. 1**

**Fig. 2**

**Fig. 3**

Fig. 4

the ratio between the surface areas of lipid and insulin

Fig. 5

Time (min)

Fig. 6

**3**

A

B

C

D

**Fig. 7**

—+—Average blood concentration of drug vs. time curve (reference formulation)

—◆—Average blood concentration of drug vs. time curve (inhalable powder)

—■—Average blood concentration of drug vs. time curve (spray)

Time (min)

**Fig. 8**

4

9A                9B

9C

**Fig.    9**

Diameter (nm)

**Fig.    10**

5

Fig. 11

Fig. 12

Fig. 13

Fig. 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 01801146
- CN 02804546
- CN 200480041300
- CN 200510028990
- CN 200510028991

### Non-patent literature cited in the description

- *The peptides Analysts, synthesis, Biology,* 1981, vol. 4, 63
- **ZHENG Shaohui ; DENG Yihui.** Application of dynamic light scattering on the evaluation of the particles size and distribution of submicroemulsion for injection. *Chinese Journal of Pharmaceutics,* 2005, vol. 3 (3), 126